# EUROPÄISCHE PATENTSCHRIFT

(11) **EP 0 403 988 B1**
(45) Veröffentlichungstag und Bekanntmachung des Hinweises auf die Patenterteilung: **25.01.1995**
(21) Anmeldenummer: 90111396.9
(22) Anmeldetag: 16.06.1990
(51) Int. Cl.: G01N 3/08, G01N 33/36

(54) **Vorrichtung zur Bestimmung von Festigkeitseigenschaften von langgestrecktem, textilem Prüfgut**
Device for determining strength characteristics of elongated textile material samples
Dispositif pour la détermination des propriétés mécaniques d'un échantillon textile allongé

(30) Priorität: 20.06.1989 CH 2292/89
(43) Veröffentlichungstag der Anmeldung: 27.12.1990
(73) Patentinhaber: ZELLWEGER LUWA AG, CH-8610 Uster (CH)
(72) Erfinder: Etter, Heinz, CH-8400 Winterthur (CH)

(56) Entgegenhaltungen:
- EP-A- 0 240 074
- SOVIET INVENTIONS ILLUSTRATED, Woche 8905, 15. März 1989, SO3, Nr. 89-037969/05, Derwent Publications Ltd, Londen, GB; & SU-A-1411 625

## Beschreibung

Die vorliegende Erfindung betrifft eine Vorrichtung zur Bestimmung von Festigkeitseigenschaften von langgestrecktem, textilem Prüfgut, mit Abzugsmitteln zum kontinuierlichen Abzug des Prüfguts von einem Vorrat, mit zwei voneinander beabstandeten, je eine rotierbare erste Walze aufweisenden, antreibbaren Klemmorganen zur Dehnung des zugeführten Prüfguts mittels Rotation der Walzen, und mit einem zwischen den Abzugsmitteln und dem in Laufrichtung des Prüfguts ersten Klemmorgan angeordneten Speicher.

Bei einer aus der EP-A-241 894 bekannten Vorrichtung dieser Art weist jedes Klemmorgan ein Stahlband auf, welches die Walze zu 180° umschlingt. Das Prüfgut wird in den Spalt zwischen Stahlband und Walze eingelegt und durch Rotation von Walze und Stahlband zwischen diesen festgeklemmt, wobei die Klemmlänge maximal den halben Umfang der Walze erreicht.

Mit dieser Vorrichtung wird erstmals eine kontinuierliche Prüfung der Festigkeitseigenschaften ermöglicht, indem das Prüfgut von seinem Vorrat kontinuierlich abgezogen und den beim Prüfvorgang angetriebenen Klemmorganen intermittierend zugeführt wird. Dadurch wird eine Prüfgeschwindigkeit erreicht, die bezogen auf den Prüfgutdurchlauf bei konventionellen Zugprüfanlagen wie beim USTER TENSORAPID (USTER = eingetragenes Warenzeichen der Zellweger Uster AG), um zwei Grössenordnungen höher liegt.

Die Prüfung der Festigkeitseigenschaften, das sind Höchstzugkraft (Reissfestigkeit) und Dehnung, war lange Zeit durch eine Vielzahl von nationalen und internationalen Normen geregelt. 1976 wurde die neue Norm DIN 53834 eingeführt, welche nur noch das sogenannte CRE-Prinzip (CRE = Constant Rate of Elongation, oder mit anderen Worten, konstante Verformungsgeschwindigkeit) zulässt. Dazu wird auf die Publikation USTER News Bulletin Nr. 26, November 1978 "USTER Prüfverfahren für das leistungsstarke Textillabor", Seite 32 ff, verwiesen.

Die in der EP-A-241 894 beschriebene Vorrichtung hat den wesentlichen Nachteil, dass sie keine Prüfung nach dem CRE-Prinzip ermöglicht, indem nämlich die Verformungsgeschwindigkeit stark von der Dehnung abhängt. Und zwar ist das Geschwindigkeitsprofil sinusförmig; die Verformungsgeschwindigkeit steigt von Null beim Festklemmen des Prüfguts bis zu einem Höchstwert bei 90° des Umschlingungswinkels an den Walzen an und erreicht dann bei 180° wieder den Wert Null. Andere nachteilige Eigenschaften dieser bekannten Vorrichtung bestehen darin, dass schon geringe Verschmutzungen zwischen Walze und Stahlband die Messgenauigkeit beeinträchtigen, und dass das Klemmprinzip keine grossen Zugkräfte zulässt. Letzteres bedeutet eine Einschränkung bezüglich des Prüfguts.

Durch die Erfindung soll nun eine Vorrichtung zur Bestimmung von Festigkeitseigenschaften von langgestrecktem, textilem Prüfgut angegeben werden, mit welcher nach dem CRE-Prinzip gemessen werden kann, und welche unempfindlich gegen Verschmutzungen und für möglichst jede Art von Prüfgut verwendbar ist.

Diese Aufgabe wird erfindungsgemäss dadurch gelöst, dass jedes Klemmorgan durch ein Walzenpaar gebildet ist, das aus der genannten ersten Walze und aus einer weiteren Walze besteht, welche letztere an ihrem Mantel abgestuft ausgebildet ist und zwei Umfangsteile mit verschiedenen Radien aufweist, dass die beiden ein Walzenpaar bildenden Walzen mit konstantem gegenseitigem Achsabstand angeordnet sind und die weitere Walze abwechselnd mit einem ersten Umfangsteil an der ersten Walze anliegt oder mit einem zweiten Umfangsteil von dieser beabstandet ist, so dass das Klemmorgan einen periodisch sich öffnenden und schliessenden Klemmspalt für das Prüfgut aufweist, und dass die Walzen mit dem abgestuften Mantel mit einem gemeinsamen Antrieb verbunden sind, der die Walzen gegensinnig antreibt, und zwar derart, dass beide Walzenpaare synchron laufen und zur gleichen Zeit klemmen oder öffnen.

Bei einer in der SU-A-1 411 625 (zitiert nach Soviet Inventions Illustrated, Woche 8905, 15. März 1989, Nr. 89-037969/05; Derwent Publications Ltd.) beschriebenen Einrichtung zur dynamischen Festigkeitsmessung von Proben werden die Proben an zwei angetriebenen Stangen eingespannt, welche jede von einer Walze mit einem abgestuften Mantel angetrieben ist. Beide Walzen sind mit einem gemeinsamen Antrieb verbunden und gegensinnig angetrieben, und zwar so, dass das Klemmen der Stangen abwechselnd erfolgt. Auf diese Weise ist die Probe einer Wechselbelastung unterworfen.

Bei einer in der EP-A-0 240 074 beschriebenen Vorrichtung zur kontinuierlichen Prüfung von grossen Garnlängen durchläuft das Prüfgut zwei gleichsinnig rotierende Klemmorgane, die durch je eine Walze und ein Anpreßelement, welches aus einem über zwei Rollen lausenden Riemen besteht, gebildet sind. Das in Transportrichtung des Prüfguts vordere Klemmorgan ist schneller angetrieben, so dass das Prüfgut einer bestimmten Dehnung ausgesetzt ist. Ein Detektor registriert die Anzahl der Fadenbrüche.

Durch die erfindungsgemässen Merkmale wird das CRE-Prinzip erfüllt, die Bestimmung der Festigkeitseigenschaften, wie Höchstzugkraft und Dehnung, erfolgt also nach dem Prinzip der konstanten Verformungsgeschwindigkeit. Dies deswegen, weil bei der Klemmung durch das Walzenpaar das Prüfgut an zwei örtlich stationären Berührungslinien zwischen den beiden Walzen festgeklemmt ist. Die Verschmutzungsgefahr ist bei dieser Klemmung wesentlich geringer als bei Klemmung zwischen Walzen und Stahlband, und bezüglich des Prüfgutmaterials bestehen praktisch keine Einschränkungen.

Ausserdem ist das Walzenpaar wesentlich weniger verschleissanfällig als die Kombination Walze-Stahlband, und die ganze Vorrichtung wird auch wesentlich kompakter. Es sind weniger Einzelteile vorhanden, und es ergibt sich infolge des Wegfalls des Stahlbandes eine Erhöhung der Sicherheit.

Im folgenden wird die Erfindung anhand eines Ausführungsbeispiels und der Zeichnungen näher erläutert; es zeigt:
- Fig. 1: eine schematische Vorderansicht einer erfindungsgemässen Vorrichtung,
- Fig. 2: Diagramme zur Funktionserläuterung; und
- Fig. 3: eine Darstellung einer eine erfindungsgemässe Vorrichtung enthaltenden Prüfanlage.

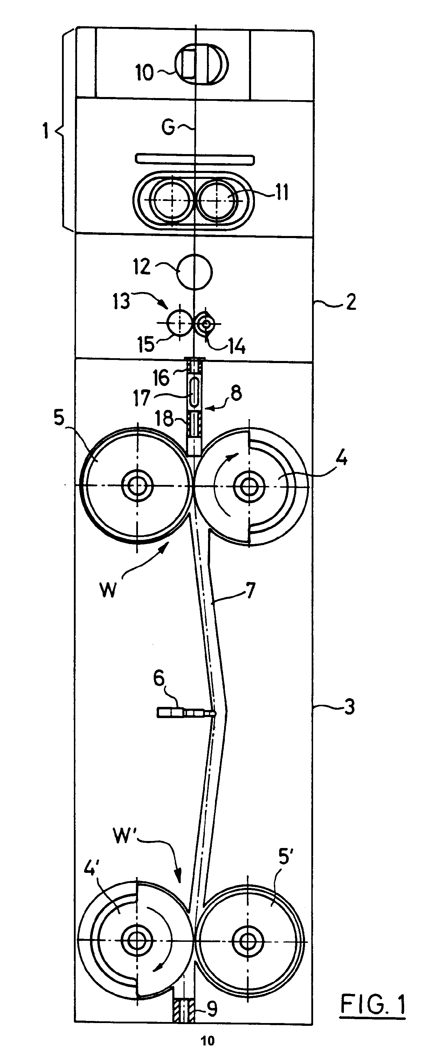

Gemäss Fig. 1 besteht die Dehnungs- und Zugkraftprüfeinrichtung im wesentlichen aus einem Zuführteil 1 für das zu prüfende Garn G, aus einem Speicherteil 2 und aus dem eigentlichen Prüfteil 3. Jeder dieser Teile kann als Modul ausgebildet sein. Der Prüfteil enthält zwei beabstandete Walzenpaare W und W′, welche je aus einer antreibbaren Transportwalze 4, 4′ und aus einer Anpresswalze 5, 5′ bestehen. Zwischen den Walzenpaaren W, W′ verläuft ein einen Kraftsensor 6 enthaltender, leicht geknickter Garnkanal 7, wobei der Kraftsensor 6 im Knickpunkt oder Scheitel des Garnkanals 7 angeordnet ist. Die beiden zueinander geneigten Teile des Garnkanals 7 sind gleich lang, so dass auf dem Sensor 6 keine Relativbewegung entsteht. Schliesslich enthält der Prüfteil 3 noch eine Einlaufpartie 8 und eine Auslaufpartie 9 für das zu prüfende Garn G.

Wesentlicher Bestandteil des Prüfteils 3 sind die beiden Walzenpaare W und W′, welche dazu dienen, eine zugeführte Garnlänge festzuklemmen und dann bei konstanter Verformungsgeschwindigkeit bis zum Bruch zu belasten. Dazu muss jedes Walzenpaar W, W′ in der Lage sein, zum Festklemmen und Freigeben der Garnlänge eine Oeffnungs- und Schliessbewegung in der Art einer üblichen Fadenklemme durchzuführen. Diese liesse sich beispielsweise durch eine gesteuerte Vergrösserung und Verkleinerung des Achsabstands der beiden Walzen 4, 5 und 4′, 5′ jedes Walzenpaares W bzw. W′ erreichen.

Gemäss Fig. 1 wird dafür eine andere Lösung gewählt: Der Achsabstand der beiden Walzen ist konstant, aber eine der beiden Walzen, und zwar die als Transportwalze bezeichnete Walze 4, 4′, ist an ihrem Umfang abgestuft ausgebildet und weist zwei Umfangsteile mit verschiedenen Radien auf. Der grosse Radius entspricht dem Abstand von der Achse der Transportwalze bis zur Achse der Anpresswalze, vermindert um deren Radius; der kleine Radius ist etwas kleiner und beide Umfangsteile erstrecken sich über beispielsweise je 180° des Mantels der Transportwalze 4, 4′.

Die Transportwalzen 4, 4′ sind in der durch einen Pfeil bezeichneten Richtung gegenläufig angetrieben, vorzugsweise durch einen gemeinsamen Antrieb, und sind darstellungsgemäss zu verschiedenen Seiten des Garnkanals 7 angeordnet. Die Anpresswalzen 5, 5′, welche an ihrem Mantel mit einem hartelastischen Belag aus beispielsweise Gummi versehen sind, sind frei drehbar gelagert und benötigen keinen speziellen Antrieb. Ihr Antrieb erfolgt infolge Reibungsmitnahme durch die grössere Transportwalzenhälfte. Wenn diese Hälfte an der zugehörigen Anpresswalze 5 oder 5′ anliegt, ist das Garn G zwischen den beiden Walzen festgeklemmt (Fadenklemme zu); im anderen Fall besteht zwischen den beiden Walzen in der Verbindungsebene zwischen ihren Achsen ein Spalt (Fadenklemme offen). Beide Transportwalzen 4 und 4′ sind ständig angetrieben und laufen streng synchron.

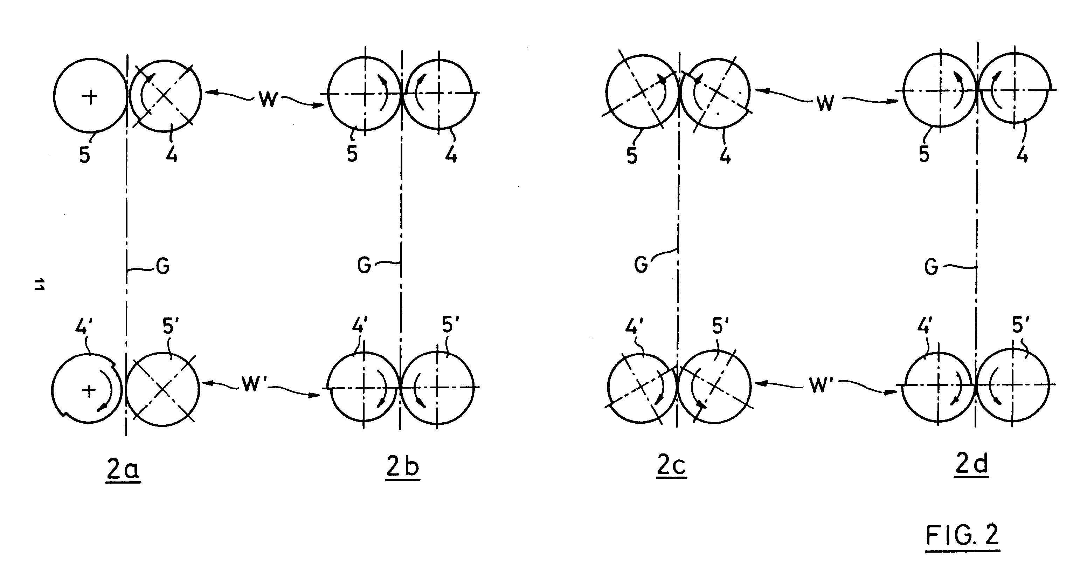

Die Vorgänge beim Klemmen, Dehnen und Freigeben eines Garns G sind in Fig. 2 anhand von 4 Diagrammen 2a bis 2d dargestellt. Jedes Diagramm zeigt schematisch die beiden Walzenpaare W und W′ mit den Walzen 4, 5 bzw. 4′, 5′ und ein Stück eines zu prüfenden Garns G; die Unterschiede zwischen den einzelnen Diagrammen liegen in der jeweiligen Drehstellung der Transportwalzen 4, 4′. Im Diagramm 2a rotiert die Transportwalzenhälfte mit dem kleineren Radius an der Anpresswalze 5, 5′ vorbei und damit dem Garn G entlang. Hier ist also der Klemmspalt offen, und das Garn G wird in den offenen Klemmspalt eingelegt.

Im Diagramm 2b erreicht gerade die Abstufung zwischen dem kleineren und dem grösseren Radius der Transportwalzen 4, 4′ die Verbindungsebene zwischen den Walzenachsen und es beginnt die Phase des gegenseitigen Kontakts zwischen den beiden Walzen 4, 5 und 4′, 5′. In diesem Moment wird das Garn G festgeklemmt und es beginnt dessen Dehnung. Im Diagramm 2c besteht immer noch Kontakt zwischen den beiden Walzen 4, 5 und 4′, 5′; der Dehnungsvorgang dauert also an.

Im Diagramm 2d erreicht gerade die Abstufung zwischen dem grösseren und dem kleineren Radius der Transportwalzen 4, 4′ die Verbindungsebene zwischen den Walzenachsen. Dies ist der Moment der grössten Dehung des Garns G, dessen Klemmung unmittelbar darauf beendet ist. Im praktischen Betrieb wird diese grösste Dehnung des Garns G nicht erreicht werden, weil dieses schon vorher gerissen ist. Oder mit anderen Worten, Dimensionierung und Betriebsparameter der Vorrichtung werden so ausgelegt, dass das jeweilige Garn G auf jeden Fall während des Dehnungsvorgangs (Diagramm 2c) reisst.

Anschliessend ist wieder der Zustand gemäss Diagramm 2a erreicht, bei welchem der abgerissene Garnteil aus der Vorrichtung entfernt und eine neue Garnlänge in diese eingelegt wird.

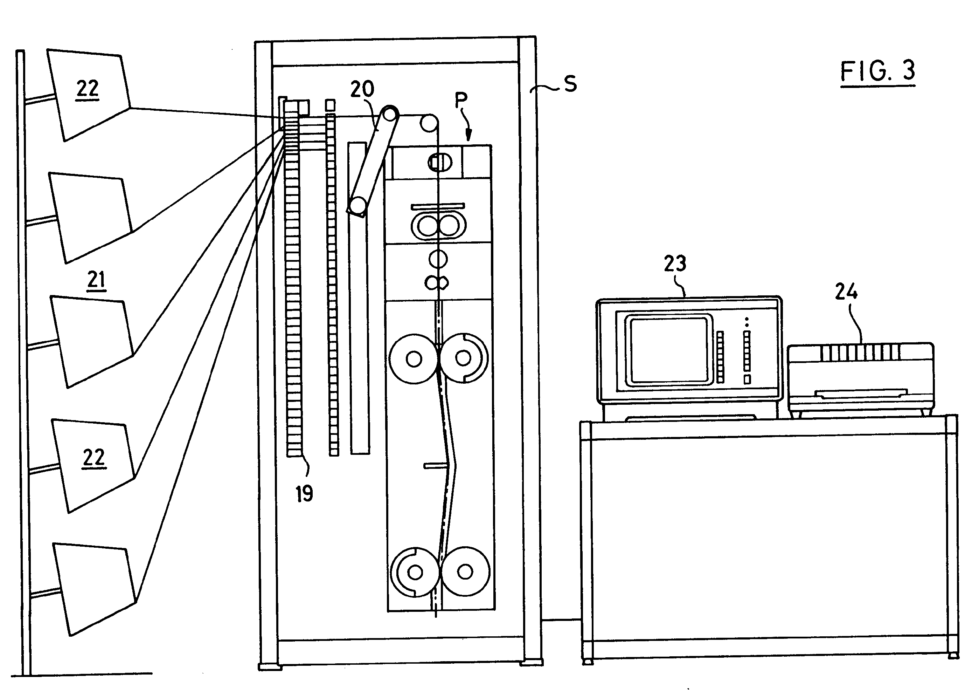

Der Zuführteil 1 der in Fig. 1 dargestellten Vorrichtung enthält im wesentlichen eine Fadenbremse 10 und ein paar motorisch antreibbare Lieferwalzen 11, mit denen das Garn G von einem Vorrat kontinuierlich abgezogen wird (siehe Fig. 3). Fadenbremse 10 und Lieferwalzen 11 sind vom USTER TESTER 3 der Zellweger Uster AG her bekannt und werden hier nicht näher beschrieben.

Wie schon erwähnt wurde, sind zwar die Transportwalzen 4, 4′ kontinuierlich angetrieben, trotzdem verlaufen aber die Verfahrensschritte Klemmen, Dehnen und Freigabe des Garns diskontinuierlich, und deswegen darf selbstverständlich während des Klemmens und während der Dehnung an das Walzenpaar W kein Garn G angeliefert werden. Aus diesem Grund ist der Speicherteil 2 vorgesehen, welcher dazu dient, das kontinuierlich angelieferte Garn G für die diskontinuierliche Auslieferung an das Walzenpaar W in einem Fadenspeicher 12 zu speichern. Ausserdem weist der Speicherteil 2 noch eine gesteuerte Fadenklemme 13 auf, welche aus einer kontinuierlich angetriebenen Steuerwalze 14 und aus einer frei drehbaren Gegenwalze 15 besteht. Die gesteuerte Fadenklemme 13 ermöglicht eine gesteuerte Leerung des Fadenspeichers 12. Die Steuerwalze 14 weist ähnlich wie die Transportwalzen 4, 4′ zwei Umfangsteile mit verschiedenen Radien auf, so dass das Garn G durch die Fadenklemme 13 entweder festgeklemmt oder freigegeben ist. Der Antrieb der Steuerwalze 14 ist mit demjenigen der Transportwalzen 4, 4′ gekoppelt; vorzugsweise ist für die Lieferwalzen 11, die Steuerwalze 14 und die Transportwalzen 4, 4′ ein einziger Antriebsmotor vorgesehen.

Die Einlaufpartie 8 des Prüfteils 3 dient zur Förderung des Garns G aus dem Fadenspeicher 12 in den Garnkanal 7 und besteht aus einer ersten Saugdüse 16, einem Zwischenspeicher 17 und einer zweiten Saugdüse 18. Prinzipiell würde eine einzige Saugdüse für diese Aufgabe genügen, dann müsste jedoch der grössere Radius der Steuerwalze 14 mit demjenigen der Transportwalze 4 übereinstimmen. Da dies nicht der Fall ist, ist ersichtlich, dass beim Abrollen des grösseren Umfangsteils der Transportwalze 4 auf der Anpresswalze 5 mehr Garn G abgezogen wird als die Steuerwalze 14 beim Abrollen ihres grösseren Umfangsteils zu liefern vermag. Deswegen ist der Zwischenspeicher 17 vorgesehen, in welchen die entsprechende Differenzlänge gespeichert wird.

Sowohl der Fadenspeicher 12 als auch der Zwischenspeicher 17 sind pneumatische Fadenspeicher der Art wie sie beispielsweise von Luftdüsenwebmaschinen her bekannt sind. Vorzugsweise sind diese Speicher durch sich von der Zeichnungsebene nach hinten erstreckende rohrförmige Kammern gebildet, welche im Bereich ihrer Eintrittsöffnung, an der das Garn G vorbeigeführt ist, eine geeignete Blas- und/oder Saugdüse zur Förderung des Garns in das Speicherrohr aufweisen. Für diese Zwecke besonders gut geeignet ist eine sogenannte Coanda-Düse der in der CH-A-672 192 (= FR-A-2 606 893) beschriebenen Art.

Der Garnkanal 7 ist durch eine in einer Grundplatte angeordnete Nut oder Rille von annähernd halbkreis- oder U-förmigem Querschnitt gebildet. Ausserdem weist die Grundplatte abgestufte Ausnehmungen zur Aufnahme der Walzenpaare W und W′ auf. An der offenen Seite des Garnkanals 7 und dieser Ausnehmungen, das ist in Fig. 1 gegen den Betrachter zu, ist der Prüfteil 3 durch eine in der Art einer Klapptüre ausgebildete, vorzugsweise durchsichtige Platte abgedeckt und abgedichtet, so dass der Garnkanal 7 und die genannten Ausnehmungen im Bereich der Walzenpaare W, W′ einen geschlossenen Luftkanal bilden. Der Kraftsensor 6 ist vorzugsweise ein piezoelektrischer Sensor.

Fig. 3 zeigt eine vollständige Zugprüfanlage mit einer Prüfeinrichtung gemäss Fig. 1. Darstellungsgemäss ist die mit P bezeichnete Prüfeinrichtung in ein schrankartiges Gehäuse S eingebaut, welches ausserdem einen Fadenwechsler 19 und einen Einlegearm 20 zum Einlegen des auf dem Fadenwechsler 19 aufgespannten Garns G in die Prüfeinrichtung P enthält. Neben dem Gehäuse S ist ein Spulengestell 21 mit Spulen 22 des zu prüfenden Garns G aufgestellt. Fadenwechsler 19, Einlegearm 20 und Spulengestell 21 sind vom USTER TENSORAPID Zugprüfgerät her bekannt.

Die Auswertung der Messignale erfolgt in einem Signalprozessor, welcher vorzugsweise in ein Bildschirmgerät 23 eingebaut ist. Dieses dient, ebenso wie ein Drucker 24 zur Resultatausgabe in numerischer und graphischer Form. Das den Signalprozessor enthaltende Bildschirmgerät 23 ist vom USTER TESTER 3 und vom USTER TENSORAPID 3 her bekannt. Ausserdem wird in diesem Zusammenhang auf die EP-A-249 741 verwiesen.

Praktische Versuche haben gezeigt, dass mit Prüfanlagen der in Fig. 3 dargestellten Art bis 400 m Garn pro Minute geprüft werden können. Somit ermöglicht also die erfindungsgemässe Vorrichtung Prüfgeschwindigkeiten, die gleich hoch sind wie diejenigen von Gleichmässigkeitsprüfern und es eröffnet sich die Möglichkeit eines vollautomatischen Textillabors mit einer vorzugsweise kombinierten Verwendung von Gleichmässigkeitsprüfer, Zugprüfanlage und Nummernbestimmung, eventuell gekoppelt mit Modulen zur Bestimmung weiterer Parameter. Ein derartiger Multitester würde dann beispielsweise aus einer Kombination der in Fig. 3 dargestellten Prüfanlage mit der in der EP-A-0 348 443 beschriebenen Vorrichtung zur Bestimmung der Gleichmässigkeit und der Feinheit bestehen.

Die erfindungsgemässe Prüfeinrichtung weist neben den schon eingangs genannten noch die folgenden Vorteile auf:
- Der konstruktive Aufbau erlaubt ein einfaches Auswechseln von Verschleissteilen, wie beispielsweise der Walzen, und eine einfache Störungsbehebung wie beispielsweise bei Verstopfungen durch kritisches Garnmaterial.
- Dadurch, dass für das Einlegen des Garns in die Walzenpaare und in die Messstrecke nur wenig Umlenkungen erforderlich sind, sind die Strömungsverluste sehr gering. Dadurch wird einerseits der Einlegevorgang wesentlich erleichtert, und andererseits kann mit weniger Energie ein grösserer Garnmaterialbereich abgedeckt werden.

## Patentansprüche

1. Vorrichtung zur Bestimmung von Festigkeitseigenschaften von langgestrecktem, textilem Prüfgut, mit Abzugsmitteln (11) zum kontinuierlichen Abzug des Prüfguts (G) von einem Vorrat (22), mit zwei voneinander beabstandeten, je eine rotierbare erste Walze (5, 5′) aufweisenden, antreibbaren Klemmorganen zur Dehnung des zugeführten Prüfguts mittels Rotation der Walzen, und mit einem zwischen den Abzugsmitteln und dem in Laufrichtung des Prüfguts ersten Klemmorgan angeordneten Speicher (12), dadurch gekennzeichnet, dass jedes Klemmorgan durch ein Walzenpaar (W, W′) gebildet ist, das aus der genannten ersten Walze (5, 5′) und aus einer weiteren Walze (4, 4′) besteht, welche letztere an ihrem Mantel abgestuft ausgebildet ist und zwei Umfangsteile mit verschiedenen Radien aufweist, dass die beiden ein Walzenpaar (W, W′) bildenden Walzen (4, 5; 4′, 5′) mit konstantem gegenseitigem Achsabstand angeordnet sind und die weitere Walze (4, 4′) abwechselnd mit einem ersten Umfangsteil an der ersten Walze (5, 5′) anliegt oder mit einem zweiten Umfangsteil von dieser beabstandet ist, so dass das Klemmorgan einen periodisch sich öffnenden und schliessenden Klemmspalt für das Prüfgut (G) aufweist, und dass die Walzen (4, 4′) mit dem abgestuften Mantel mit einem gemeinsamen Antrieb verbunden sind, der die Walzen gegensinnig antreibt, und zwar derart, dass beide Walzenpaare (W, W′) synchron laufen und zur gleichen Zeit klemmen oder öffnen.

2. Vorrichtung nach Anspruch 1, dadurch gekennzeichnet, dass der grössere Radius der weiteren Walze (4, 4′) dem Abstand zwischen den Achsen der beiden Walzen (4, 5; 4′, 5′), vermindert um den Radius der ersten Walze (5, 5′) und um die Stauchtiefe, entspricht.

3. Vorrichtung nach Anspruch 2, dadurch gekennzeichnet, dass sich die beiden Umfangsteile mit den verschiedenen Radien je über einen bestimmten Teil, vorzugsweise die Hälfte, des Umfangs der weiteren Walzen (4, 4′) erstrecken.

4. Vorrichtung nach Anspruch 2 oder 3, gekennzeichnet durch einen zwischen den Walzenpaaren (W, W′) verlaufenden Führungskanal (7) für das Prüfgut (G), in welchem ein Sensor (6) zur Kraftmessung angeordnet ist.

5. Vorrichtung nach Anspruch 4, dadurch gekennzeichnet, dass die Walzen (4, 4′) mit dem abgestuften Mantel zu verschiedenen Seiten des Führungskanals (7) angeordnet sind.

6. Vorrichtung nach Anspruch 4, dadurch gekennzeichnet, dass der Führungskanal (7) aus zwei zueinander geneigten, gleich langen Teilen besteht, und dass der Sensor (6) im Uebergangsbereich zwischen diesen beiden Teilen angeordnet ist.

7. Vorrichtung nach Anspruch 2, dadurch gekennzeichnet, dass der Speicher durch einen pneumatischen Speicher (12) gebildet ist.

8. Vorrichtung nach Anspruch 7, gekennzeichnet durch pneumatische Mittel für den Transport des Prüfguts (G) von den Abzugsmitteln (11) zum ersten Walzenpaar (W).

9. Vorrichtung nach Anspruch 8, dadurch gekennzeichnet, dass zwischen dem Speicher (12) und dem ersten Walzenpaar (W) eine gesteuerte Fadenklemme (13) angeordnet ist.

10. Vorrichtung nach Anspruch 9, dadurch gekennzeichnet, dass die gesteuerte Fadenklemme (13) durch ein aus einer Steuerwalze (14) und aus einer Gegenwalze (15) bestehendes Walzenpaar gebildet ist.

11. Vorrichtung nach Anspruch 10, dadurch gekennzeichnet, dass die Steuerwalze (14) zwei Umfangsteile mit verschiedenen Radien aufweist, und dass das Prüfgut (G) bei Anliegen am Umfangsteil mit dem grösseren Radius festgeklemmt und bei Anliegen am Umfangsteil mit dem kleineren Radius freigegeben ist.

12. Vorrichtung nach Anspruch 11, dadurch gekennzeichnet, dass die Steuerwalze (14) einen Antrieb aufweist, welcher mit dem Antrieb der Walzenpaare (W, W′) verbunden ist.

13. Vorrichtung nach Anspruch 12, gekennzeichnet durch einen gemeinsamen Antrieb für die Walzenpaare (W, W′), die Steuerwalze (14) und die Abzugsmittel (11).

14. Vorrichtung nach Anspruch 11, dadurch gekennzeichnet, dass zwischen der Steuerwalze (14) und dem ersten Walzenpaar (W) ein Einlaufteil (8) und nach dem zweiten Walzenpaar (W′) ein Auslaufteil (9) für das Prüfgut (G) vorgesehen ist.

15. Vorrichtung nach Anspruch 14, dadurch gekennzeichnet, dass der Einlaufteil (8) zwei Düsen (16, 18) und einen zwischen diesen angeordneten Zwischenspeicher (17) umfasst.

16. Vorrichtung nach Anspruch 15, dadurch gekennzeichnet, dass die Düsen (16, 18) durch Saugdüsen gebildet sind, und dass der Zwischenspeicher (17) durch einen pneumatischen Speicher gebildet ist.

17. Vorrichtung nach Anspruch 16, dadurch gekennzeichnet, dass der Speicher (12) und der Zwischenspeicher (17) einen senkrecht zur Achse des Prüfguts (G) angeordneten, rohrförmigen Speicherraum mit einer Eintrittsöffnung für das Prüfgut aufweisen, und dass das Prüfgut an dieser Eintrittsöffnung vorbeigeführt ist.

18. Vorrichtung nach Anspruch 17, dadurch gekennzeichnet, dass im Bereich der Eintrittsöffnung des Speichers (12) und des Zwischenspeichers (17) je eine Coanda-Düse angeordnet ist.

19. Vorrichtung nach Anspruch 14, dadurch gekennzeichnet, dass der Auslaufteil (9) eine Saugdüse enthält.

## Claims

1. Apparatus for determining strength properties of long, textile test material, comprising drawing-off means (11) for continuously drawing-off the test material (G) from a supply (22), two driveable clamping members, at a distance from one another and each having a rotatable first roller (5, 5′), for elongating the fed test material by means of rotation of the rollers, and a store (12) arranged between the drawing-off means and the first clamping member in the running direction of the test material, characterized in that each clamping member is formed by a roller pair (W, W′), which comprises the abovementioned first roller (5, 5′) and a further roller (4, 4′), the latter being of a stepped configuration on its circumferential surface and having two peripheral parts of different radii, in that the two rollers (4, 5; 4′, 5′) which form a roller pair (W, W′) are arranged with a constant mutual distance between the axes, and the further roller (4, 4′) alternately bears against the first roller (5, 5′) with a first peripheral part or is spaced apart from said first roller with a second peripheral part, with the result that the clamping member has a periodically opening and closing clamping gap for the test material (G), and in that the rollers (4, 4′) with the stepped circumferential surface are connected to a common drive which drives the rollers in opposite directions, to be precise such that the two roller pairs (W, W′) run synchronously and effect a clamping or opening action at the same time.

2. Apparatus according to Claim 1, characterized in that the larger radius of the further roller (4, 4′) corresponds to the distance between the axes of the two rollers (4, 5; 4′, 5′) reduced by the radius of the first roller (5, 5′) and by the compressed depth.

3. Apparatus according to Claim 2, characterized in that the two peripheral parts having the different radii each extend over a certain part of the periphery, preferably half the periphery, of the further rollers (4, 4′).

4. Apparatus according to Claim 2 or 3, characterized by a guide channel (7), running between the roller pairs (W, W′), for the test material (G), in which guide channel (7) a sensor (6) for the measurement of force is arranged.

5. Apparatus according to Claim 4, characterized in that the rollers (4, 4′) having the stepped circumferential surface are arranged on different sides of the guide channel (7).

6. Apparatus according to Claim 4, characterized in that the guide channel (7) consists of two parts which are inclined towards one another and are of the same length, and in that the sensor (6) is arranged in the transition area between these two parts.

7. Apparatus according to Claim 2, characterized in that the store is formed by a pneumatic store (12).

8. Apparatus according to Claim 7, characterized by pneumatic means for the transport of the test material (G) from the drawing-off means (11) to the first roller pair (W).

9. Apparatus according to Claim 8, characterized in that a controlled thread clamp (13) is arranged between the store (12) and the first roller pair (W).

10. Apparatus according to Claim 9, characterized in that the controlled thread clamp (13) is formed by a roller pair consisting of a control roller (14) and a mating roller (15).

11. Apparatus according to Claim 10, characterized in that the control roller (14) has two peripheral parts of different radii, and in that the test material (G) is clamped in place when bearing against the peripheral part having a larger radius and is released when bearing against the peripheral part having the smaller radius.

12. Apparatus according to Claim 11, characterized in that the control roller (14) has a drive which is connected to the drive of the roller pairs (W, W′).

13. Apparatus according to Claim 12, characterized by a common drive for the roller pairs (W, W′), the control roller (14) and the drawing-off means (11).

14. Apparatus according to Claim 11, characterized in that, for the test material (G), an entry part (8) is provided between the control roller (14) and the first roller pair (W), and an exit part (9) is provided after the second roller pair (W′).

15. Apparatus according to Claim 14, characterized in that the entry part (8) comprises two nozzles (16, 18) and an intermediate store (17) arranged between them.

16. Apparatus according to Claim 15, characterized in that the nozzles (16, 18) are formed by suction nozzles, and in that the intermediate store (17) is formed by a pneumatic store.

17. Apparatus according to Claim 16, characterized in that the store (12) and the intermediate store (17) have a tubular storage space which is arranged perpendicularly to the axis of the test material (G) and has an inlet opening for the test material, and in that the test material is directed past this inlet opening.

18. Apparatus according to Claim 17, characterized in that one Coanda nozzle each is arranged in the area of the inlet opening of the store (12) and the intermediate store (17).

19. Apparatus according to Claim 14, characterized in that the exit part (9) contains a suction nozzle.

## Revendications

1. Dispositif pour déterminer des propriétés mécaniques d'un échantillon textile allongé, avec des moyens de tirage (11) pour le déroulement continu de l'échantillon (G) d'un dispositif d'alimentation (22), avec deux organes de serrage espacés l'un de l'autre, présentant chacun un premier rouleau tournant (5, 5′) et pouvant être entraînés pour l'allongement de l'échantillon amené au moyen de la rotation des rouleaux, et avec un dispositif de stockage (12) disposé entre les moyens de tirage et le premier organe de serrage dans le sens de déplacement de l'échantillon, caractérisé en ce que chaque organe de serrage est formé par une paire de rouleaux (W, W′), qui est constituée du premier rouleau cité (5,5′) et d'un rouleau supplémentaire (4,4′) dont l'enveloppe est réalisée à gradins et qui présente deux parties de pourtour avec des rayons différents, en ce que les deux rouleaux (4, 5; 4′, 5′) formant une paire de rouleaux (W, W′) sont disposés suivant une distance d'axe-en-axe mutuelle constante, et que le rouleau supplémentaire (4, 4′) s'applique alternativement par une première partie de pourtour au premier rouleau (5, 5′) ou se trouve à une distance de celui-ci par une deuxième partie de pourtour, de façon que l'organe de serrage présente une fente de serrage s'ouvrant et se fermant périodiquement pour l'échantillon (G), et en ce que les rouleaux (4, 4′) pourvus de l'enveloppe à gradins sont reliés à une commande commune qui entraîne les rouleaux en sens inverse et cela de façon que les deux paires de rouleaux (W,W′) tournent de manière synchrone et procèdent en même temps au serrage ou à l'ouverture.

2. Dispositif selon la revendication 1, caractérisé en ce que le rayon plus grand du rouleau supplémentaire (4, 4′) correspond à la distance entre les axes des deux rouleaux (4, 5; 4′, 5′), diminué du rayon du premier rouleau (5, 5') et de la profondeur de compression.

3. Dispositif selon la revendication 2, caractérisé en ce que les deux parties de pourtour avec les rayons différents s'étendent respectivement sur une partie déterminée, de préférence la moitié du pourtour des rouleaux supplémentaires (4, 4′).

4. Dispositif selon la revendication 2 ou 3, caractérisé par un canal de guidage (7) s'étendant entre les paires de rouleaux (W, W′) pour l'échantillon (G), et dans lequel est disposé un capteur (6) pour la mesure de force.

5. Dispositif selon la revendication 4, caractérisé en ce que les rouleaux (4, 4′) pourvus de l'enveloppe à gradins sont disposés aux côtés opposés du canal de guidage (7).

6. Dispositif selon la revendication 4, caractérisé en ce que le canal de guidage (7) est constitué de deux parties de même longueur inclinées l'une vers l'autre, et en ce que le capteur (6) est disposé dans la zone de transition entre ces deux parties.

7. Dispositif selon la revendication 2, caractérisé en ce que le dispositif de stockage est constitué par un dispositif de stockage pneumatique (12).

8. Dispositif selon la revendication 7, caractérisé par des moyens pneumatiques pour le transport de l'échantillon (G) des moyens de tirage (11) à la première paire de rouleaux (W).

9. Dispositif selon la revendication 8, caractérisé en ce qu'il est disposé entre le dispositif de stockage (12) et la première paire de rouleaux (W) un pince-fil (13) commandé.

10. Dispositif selon la revendication 9, caractérisé en ce que le pince-fil commandé (13) est constitué par une paire de rouleaux comprenant un rouleau de commande (14) et un contre-rouleau (15).

11. Dispositif selon la revendication 10, caractérisé en ce que le rouleau de commande (14) présente deux parties de pourtour avec des rayons différents, et en ce que l'échantillon (G) lors de l'application à la partie de pourtour présentant le rayon plus grand est serré et lors de l'application à la partie de pourtour présentant le rayon plus petit est libéré.

12. Dispositif selon la revendication 11, caractérisé en ce que le rouleau de commande (14) présente un dispositif d'entraînement qui est relié au dispositif d'entraînement des paires de rouleaux (W, W′).

13. Dispositif selon la revendication 12, caractérisé par un dispositif d'entraînement commun pour les paires de rouleaux (W, W′), le rouleau de commande (14) et les moyens de tirage (11).

14. Dispositif selon la revendication 11, caractérisé en ce qu'il est prévu entre le rouleau de commande (14) et la première paire de rouleaux (W) une partie d'entrée (8) et derrière la deuxième paire de rouleaux (W') une partie de sortie (9) pour l'échantillon (G).

15. Dispositif selon la revendication 14, caractérisé en ce que la partie d'entrée (8) comprend deux buses (16, 18) et un dispositif de stockage intermédiaire (17) disposé entre celles-ci.

16. Dispositif selon la revendication 15, caractérisé en ce que les buses (16, 18) sont constituées par des buses d'aspiration, et que le dispositif de stockage intermédiaire (17) est constitué par un dispositif de stockage pneumatique.

17. Dispositif selon la revendication 16, caractérisé en ce que le dispositif de stockage (12) et le dispositif de stockage intermédiaire (17) présentent une enceinte de stockage tubulaire disposée perpendiculairement à l'axe de l'échantillon (G) avec une ouverture d'entrée pour l'échantillon, et en ce qu'on fait passer l'échantillon devant cette ouverture d'entrée.

18. Dispositif selon la revendication 17, caractérisé en ce qu'une buse Coanda est disposée respectivement au voisinage de l'ouverture d'entrée du dispositif de stockage (12) et du dispositif de stockage intermédiaire (17).

19. Dispositif selon la revendication 14, caractérisé en ce que la partie de sortie (9) comprend une buse d'aspiration.
